# EUROPÄISCHE PATENTANMELDUNG

(11) **EP 0 766 954 A1**
(43) Veröffentlichungstag der Anmeldung: **09.04.1997**
(21) Anmeldenummer: 96115460.6
(22) Anmeldetag: 26.09.1996
(51) Int. Cl.: A61H 7/00, A61F 7/00

(54) **Therapeutisches Kopfband**

(30) Priorität: 02.10.1995 DE 19536731
(71) Anmelder: Kirschke, Judit, 76297 Stutensee-Blankenloch (DE)
(72) Erfinder: Kirschke, Judit, 76297 Stutensee-Blankenloch (DE)
(74) Vertreter: Zahn, Roland, Dipl.-Ing.

(57) **Zusammenfassung**

Das therapeutische Kopfband besteht ausschließlich aus natürlichen und weitgehend naturbelassenen Materialien und ist wenigstens abschnittsweise als Kissen (2) ausgebildet. Dieses Kissen (2) ist mit im wesentlichen kugelförmigen Naturstoffen zur Erzeugung einer Massagewirkung gefüllt. An einer oder beiden Oberflächen (16) des Kissens ist eine Tasche ausgebildet, in der eine oder mehrere Einlagen (30) auswechselbar angeordnet sind.
Das Kopfband ist insbesondere zur Behandlung von Migränekopfschmerzen, zur Behandlung von Ohrenentzündungen und zur Behandlung von Nasen- und/oder Nasennebenhöhlenerkrankungen sowie als Kompresse zur Behandlung von Nackenschmerzen vorgesehen..

## Beschreibung

Die Erfindung betrifft ein therapeutisches Kopfband.

Ein solches Kopfband wird insbesondere bei der Behandlung von Kopf- , Ohren- oder Nasen- bzw. Nasennebenhöhlenschmerzen eingesetzt. Daneben sind aber auch andere Verwendungsmöglichkeiten denkbar, beispielsweise als Kompresse zur Behandlung von Nackenschmerzen.

Aus dem Stand der Technik sind vor allem sogenannte Migränebänder bekannt. Migräne ist ein weit verbreitetes Leiden, das die betroffenen Personen sehr stark beeinträchtigt, dessen Ursachen auch heute noch nicht vollständig bekannt sind und für das es auch heute noch keine allgemeine zufriedenstellende Behandlungsmethode gibt. Nach dem heutigen Wissensstand sind die Migräneschmerzen zum großen Teil auf Gefäßveränderungen in Form von kurzzeitigen Konstriktionen und anschließenden Dilatationen zurückzuführen. Die derzeit auf dem Markt befindlichen Medikamente zur Behandlung von Migräne sind deshalb hauptsächlich auf die Konstriktion von Blutgefäßen ausgerichtet. Diese Medikamente helfen jedoch nicht immer und haben überdies oft unangenehme Nebenwirkungen, weshalb viele Migränepatienten eine alternative Behandlung ohne Medikamenteneinnahme vorziehen. Zu diesen alternativen Behandlungsverfahren gehören die Druck- und Massagebehandlung, die Wärme- und Kältebehandlung, die Behandlung mit ätherischen Ölen und die Aromatherapie.
Zu jedem dieser Behandlungsverfahren gibt es zahlreiche Ausführungsformen und Hilfsmittel, aber eine Vorrichtung bzw. ein Mittel, mit der bzw. dem alle diese Behandlungsverfahren gleichzeitig durchgeführt werden können, ist bisher noch nirgends beschrieben.
Aus der US Patentschrift 4 944 289 ist zwar ein therapeutisches Kopfband bekannt, das aus einem schlauchförmigen Grundkörper besteht, der um den Kopf gebunden wird, und in dem an ganz bestimmten Stellen rollen- bzw. tonneförmige Druckkörper angeordnet sind, die mit ihrer einen Stirnfläche auf den Kopf des Trägers einwirken, wobei der Druck durch die Spannung des umgebundenen Grundkörpers erzeugt wird. Eine gleichzeitige Wärmebehandlung, Aromabehandlung oder ähnliche ergänzende Kopfschmerzbehandlung ist mit diesem Kopfband jedoch nicht möglich und wird in der US-Schrift auch gar nicht in Erwägung gezogen. Außerdem ist dieses Kopfband nur für eine Preß-Druck-Massage einsetzbar, da die verwendeten Druckkörper nur auf einige wenige, relativ großflächige Punkte am Kopf des Trägers einen permanenten Druck mit konstanter Stärke ausüben.

Es ist deshalb eine Aufgabe der vorliegenden Erfindung, eine Vorrichtung bzw. ein Mittel bereitzustellen, mit der bzw. dem sich die genannten Verfahren zur Behandlung von Migränekopfschmerzen, aber auch von anderen Kopfschmerzen und sonstigen Schmerzen im Kopfbereich, insbesondere auch Ohren- und Nasennebenhöhlenschmerzen gleichzeitig und beliebig kombiniert durchführen lassen, das deshalb eine besonders gute Heilwirkung aufweist und das einfach, schnell und kostengünstig hergestellt werden kann.

Eine Lösung dieser Aufgabe besteht in der Bereitstellung eines therapeutischen Kopfbandes, das ausschließlich aus natürlichen und weitgehend naturbelassenen Materialien besteht und wenigstens abschnittsweise als Kissen ausgebildet ist, wobei dieses Kissen mit im wesentlichen kugelförmigen Naturstoffen gefüllt ist, die einen Massageeffekt erzeugen. An einer oder beiden Oberflächen des Kissens ist außerdem eine Tasche ausgebildet, in der eine oder mehrere Einlagen auswechselbar angeordnet sind.
Der Begriff "weitgehend naturbelassen" bedeutet in diesem Zusammenhang, daß die betreffenden Materialien zwar ganz überwiegend, aber unter Umständen doch nicht vollständig naturbelassen -weil z.B. gefärbt- sind.

Das erfindungsgemäße Kopfband ermöglicht dem Patienten, mehrere Heil- und Linderungsverfahren zu kombinieren, insbesondere Druck- und Massagebehandlung, Wärme- und Kältebehandlung, Inhalationsbehandlung und Aromatherapie. Je nach Schmerzart und individuellen Empfindlichkeiten kann auf einfachste Art und Weise, schnell und ohne großen Aufwand eine Heilbehandlung durchgeführt werden, die auf die ganz persönlichen Umstände und Bedürfnisse des Patienten abgestimmt ist und die auch deshalb besonders wirkunsvoll ist.

Eine vorteilhafte Weiterbildung der Erfindung sieht vor, daß die Naturstoffe Wärme und/oder Kalte speichern können. Diese Ausführungsform eignet sich besonders gut für eine kombinierte Massage und Kälte- bzw Wärmebehandlung, beispielsweise bei Migränekopfschmerzen.

Um eine Wärmetherapie u.U. mit relativ hohen Temperaturen über einen längeren Zeitraum zu gewährleisten, sollten die Naturstoffe beständig gegen Hitze von bis zu 150°C sein.

Als Naturstoffüllung tur das Kissen sind vorzugsweise Hirseschalen vorgesehen. Diese Hirseschalen üben einen feinen Druck- und Massageeffekt aus. Sie können darüberhinaus problemlos auch auf hohe Temperaturen von bis zu 150°C erhitzt werden und speichern eine vorgegebene Temperatur relativ lange. Für eine Langzeitbehandlung mit relativ hoher Wärme sollten die Hirseschalen mit Torf kombiniert werden. Es wurde nämlich überraschenderweise gefunden, daß eine Kombination von Hirseschalen und Torf die Wärme besonders lange hält. Die Mischung aus Torfund Hirseschalen laßt sich sehr schnell und auf mindestens 150°C erhitzen (beispielsweise in einem handelsüblichen Küchenofen), ohne dabei zu entflammen, und speichert die Wärme über mehrere Stunden. Darüberhinaus haben Torf und Hirse bekanntermaßen eine beruhigende Wirkung, und Torfbesitzt zudem aufgrund seines Huminsäuregehalts keimtötende Eigenschaften.

Auch Kirschkerne kommen als Kissenfüllung in Betracht. Sie lassen sich ebenfalla problemlos auf relativ hohe Temperaturen (beispielsweise 150°C) erhitzt werden, ohne dabei zu entflammen. Andererseits ist jedoch bei Kirschkernen ein Anwärmen nicht unbedingt notwendig, da - wie überraschenderweise gefunden wurde - Kirschkerne die Körperwärme relativ schnell aufnehmen und speichern können. (Bereits nach etwa 1 Stunde können sie sich von Raumtemperatur auf Körpertemperatur erwärmt haben.)

Eine Kirschkernfüllung weist darüberhinaus die Vorteile auf, daß sie eine besonders ausgeprägte Massagewirkung entfalten kann.

Es besteht die Möglichkeit, die Kissenfüllung zu aromatisieren, beispielsweise mit Lavendel und/oder Kamille und/oder anderen Düften. Dadurch kann ein zusätzlicher Beruhigungs- und Entspannungseffekt erzielt werden.

Bei einer bevorzugten Ausführungsform ist die Tasche an der Kissenoberfläche bzw sind die Taschen an den beiden Kissenoberflächen so dimensioniert, daß sie sich über die gesamte Oberfläche(n) erstreckt bzw erstrecken. In diese Tasche(n) können zusätzliche Einlagen zur Kältebehandlung, zur Wärmebehandlung oder zur Aromatherapie eingelegt werden, die im wesentlichen die gleichen Abmessungen wie das Kissen haben und die deshalb ihre ergänzende Heilwirkung über der gesamten Kissenfläche entfalten. Damit ist eine optimale Kombinationswirkung gewährleistet.

Bei einer Variante der Erfindung ist vorgesehen, daß die Einlage(n) aus Naturfasern mit einem hohem Feuchtigkeitsaufnahmevermögen besteht (bestehen). Dieses Kopfband eignet sich besonders für Wärmetherapien, weil die Einlage(n) die sich bildende Schwitzfeuchtigkeit schnell, einfach und hygienisch aufnehmen kann (können).
Als Einlage(n) für die Kopfbandtasche kommt (kommen) außerdem ein Kissen mit einer Füllung aus aromatischem und/oder wärmespeicheindem und/oder kältespeicherndem Material in Betracht. Durch die Wahl der Einlage(n) kann das Kopfband ganz spezifisch auf die jeweilige Behandlungsart abgestimmt und seine Wirkung wesentlich verstärkt werden.

Als wärmespeicherndes Hüll- und/oder Füllmaterial eignen sich insbesondere Torf, Seide oder Wolle oder eine Kombination davon, da diese Materialien gut erhitzt werden können, ohne dabei zu entflammen und die Wärme relativ lange speichern.

Als kältespeichernde Materialien eignen sich insbesondere Seide, Hirseschalen und Kirschkerne. Diese Materialien können gut aufniedrige Temperaturen abgekühlt werden und speichern die Kälte relativ lange.

Als aromatische Materialien werden insbesondere Kräuter, Blüten oder mit aromatischen Ölen getränkte Naturfasern oder eine Kombination davon vorgeschlagen. Durch die Wahl des jeweiligen Aromastoffes kann die therapeutische Wirkung des Kopfbands gezielt beeinflußt, d.h. ergänzt oder verstärkt werden. Es können z.B. ätherische Öle eingesetzt werden, die den Stoffwechsel anregen, oder solche, die Stoffwechselabfallprodukte neutralisieren bzw. Bakterien inaktivieren, oder solche, die beruhigend und entspannend wirken.

Grundsätzlich sollten alle für das Kopfband und die Einlage(n) verwendeten Materialien so aufeinander abgestimmt sein, daß sie sich in ihren Heilwirkungen gegenseitig ergänzen bzw. verstärken. Durch die Wahl der zu kombinierenden Materialien kann somit der therapeutische Effekt des Kopfbandes gezielt variiert werden.

Aus modisch-ästhetischen Gründen kann das Kopfband mit einem farbigen Seidentuch umwickelt sein. In diesem Fall kann aufdie Bandabschnitte verzichtet werden.

Neben dem therapeutischen Kopfband als solchem umfaßt die vorliegende Erfindung auch die Verwendung desselben als Stirnkompresse, insbesondere zur Behandlung von Migränekopfschmerzen, als Ohrenkompresse, insbesondere zur Behandlung von Ohrenentzündungen, und als Nasen- und/oder Nasennebenhöhlenkompresse zur Behandlung von Nasen- und Nasennebenhöhlenerkrankungen.

Nachfolgend sind einige bevorzugte Materialien für das Kopfband und die Einlage(n) mit ihren erfindungswesentlichen Eigenschaften aufgelistet:
- Torf:: problemlos bis 150°C erhitzbar - beispielsweise im Backofen,
ausgezeichnetes Temperaturisolationsvermögen,
sehr gutes Temperaturspeichervermögen,
gutes Feuchtigkeitsaufnahme (bis zu 80% des Eigengewichts),
gutes Feuchtigkeitsabgabevermögen,
antibakterielle Wirkung,
durchblutungsfordernde Wirkung,
Heilwirkung bei Beschwerden aufgrund innerer Verspannungen
- Hirseschalen:: problemlos bis 150°C erhitzbar - beispielsweise im Backofen,
problemlos abkühlbar - beispielsweise im Gefrierschrank,
besonders gutes Temperaturspeichervermögen,
hoher Gehalt an Magnesium und Kieselsäure,
beruhigende Wirkung,
heilungsfördernde Wirkung
- Kirschkerne:: problemlos bis 150°C erhitzbar - beispielsweise im Backofen,
problemlos abkühlbar - beispielsweise im Gefrierschrank,
sehr hohes Temperaturspeichervermögen
- Wolle:: gutes Temperaturisolationsvermögen,
gutes Temperaturspeichervermögen,
gutes Feuchtigkeitsaufnahme (bis zu 30% des Eigengewichts),
gutes Feuchtigkeitsabgabevermögen,
antibakterielle Wirkung
- Seide:: besonders gut hautverträglich,
atmungsaktiv,
gutes Temperaturisolationsvermögen,
gutes Temperaturspeichervermögen,
gutes Feuchtigkeitsaufnahmevermögen (bis zu 40% des Eigengewichts),
gutes Feuchtigkeitsabgabevermögen,
beruhigende, entspannende Wirkung

Die Erfindung wird im folgenden anhand von in den Zeichnungen dargestellten Ausführungsbeispielen näher erläutert. Es zeigen
- Fig.1:: ein erfindungsgemäßes Kopfband zur Behandlung von Migräneschmerzen,
- Fig.2:: das Kopfband nach Fig. 1 längs der Schnittlinie II - II,
- Fig.3:: ein erfindungsgemäßes Kopfband zur Behandlung von Ohrenschmerzen und
- Fig.4:: ein erfindungsgemäßes Kopfband zur Behandlung von Nasennebenhöhlenerkrankungen.

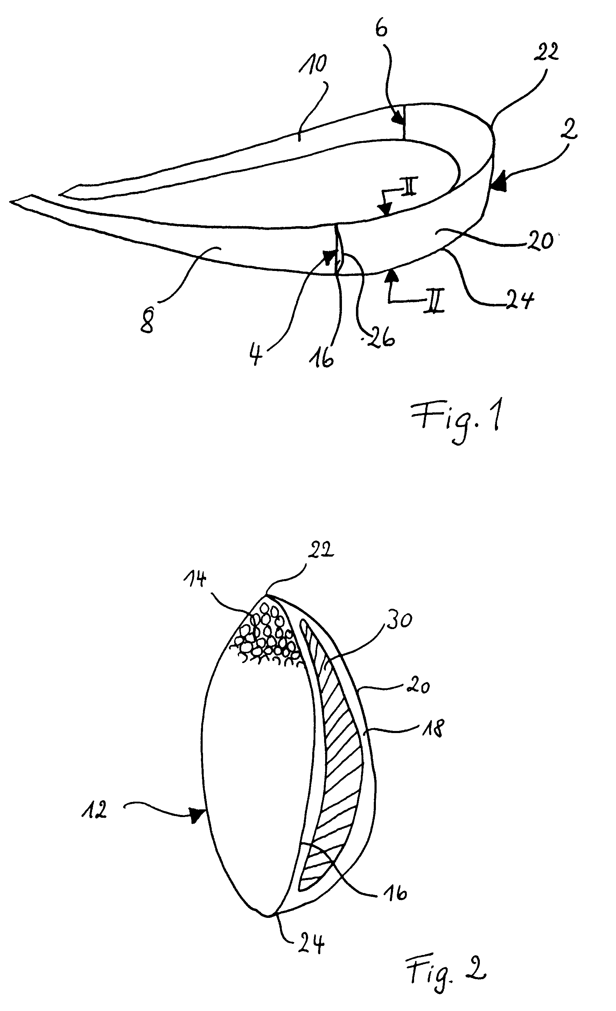

Das in Fig. 1 dargestellte Kopfband zur Behandlung von Migräneschmerzen weist einen kissenförmigen Abschnitt 2 in Form eines langgezogenen, abgeflachten Rechtecks auf, das die Stirn eines erwachsenen Menschen nahezu vollständig bedecken kann. An seinen beiden Schmalseiten 4, 6 geht dieses Kissen 2 in je einen Bandabschnitt 8, 10 über, der frei endet. Mit diesen beiden Bandabschnitten 8, 10 wird das Kopfband so am Kopf des Patienten befestigt, daß das Kissen fest an die Stirn gedrückt wird. Eine zusätzliche Heilwirkung ergibt sich dadurch, daß über das Kissen 2 bzw die Bandabschnitte 8, 10 ein leichter Druck auf die Akupressurpunkte "hsi-San" an den Schläfen und "fen-chi" am Hinterkopf des Patienten ausgeübt wird.

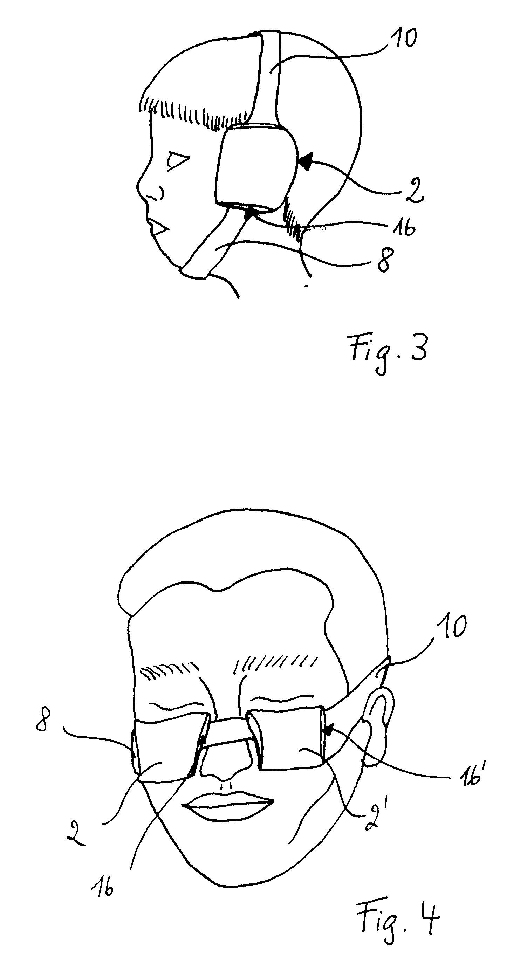

Wie aus Fig. 2 ersichtlich, besteht das Kissen aus einer Kissenhülle 12, die die Kissenfüllung 14 aus kugelförmigen Naturstoffen, vorzugsweise Hirsekörnern oder auch Kirschkernen, umgibt. Diese Naturstoffe können je nach Bedarferwärmt bzw. erhitzt oder abgekühlt werden. Die Erwärmung kann im Backofen erfolgen, die Abkühlung im Kühl- oder Gefrierschrank. An der der Stirn des Patienten abgewandten Oberfläche 16 der Kissenhülle 12 ist eine schlauchförmige Tasche 18 ausgebildet, die sich über die gesamte Kissenoberfläche 16 erstreckt. Die Taschenseitenflächen werden stirnseitig von der Kissenhülle 12 und stirnabgewandt von einem aufgesetzten Materialstreifen 20 gebildet, der an seinen Längskanten 22, 24 mit der Kissenhülle 12 verbunden ist. Im einfachsten Fall sind Tasche 18 und Kissenhülle 12 miteinander vernäht. Die beiden schmalen Stirnseiten 26, 28 der Tasche 18 sind offen, so daß von beiden Seiten eine oder mehrere Einlagen 30 in die Tasche 16 eingeschoben werden können. Die Einlagen 30 sitzen in der Tasche 16 durch Form- und/oder Reibschuß so fest, daß sie in der Regel nicht selbständig herausrutschen können. Als Einlage kommt insbesondere ein Kissen aus Seide und/oder Wolle in Betracht, das mit ätherischen Ölen getränkt bzw. auf andere Art spezifisch aromatisiert ist. Geeignete Aromastoffe sind beispielsweise Lavendel, Kamille, Pfefferminze oder Menthol.

Das beschriebene "Migränekopfband" kann auch zur Behandlung von Hals- oder Nackenschmerzen eingesetzt werden.

Zur Behandlung von Ohrenschmerzen wird vorzugsweise ein Kopfband gemäß Fig. 3 eingesetzt. Dieses Kopfband unterscheidet sich von der in Fig. 1 und Fig. 2 dargestellten Variante zum einen durch die Abmessungen des Kissens 2, die derart gewählt sind, daß das Ohr des Patienten vollständig bedeckt ist. Zum zweiten besteht die Füllung des Kissens 2 aus Hirse und Torf, um eine längere, konstante Wärmebehandlung zu gewährleisten die gegenüber dem Massageeffekt im Vordergrund steht.

Das beschriebene "Ohrenkopfband" kann ebensogut zur Behandlung von Nasenleiden verwendet werden.
In Fig. 4 ist eine Ausführungsform des erfindungsgemäßen Kopfbands dargestellt, die zur Behandlung von Nasennebenhöhlenerkrankungen vorgesehen ist. Diese Ausführungsform unterscheidet sich von der in Fig. 1 und Fig. 2 dargestellten Variante dadurch, daß zwei Kissen 2, 2' im Abstand einer Nasenbreite voneinander angeordnet sind, die die Nasennebenhöhlenbereiche im wesentlichen bedecken.

### Bezugszeichen

- 2: kissenförmiger Abschnitt, Kissen
- 4: Schmalseite
- 6: Schmalseite
- 8: Bandabschnitt
- 10: Bandabschnitt
- 12: Kissenhülle
- 14: Kissenfüllung
- 16: Oberfläche der Kissenhülle
- 18: Tasche
- 20: Materialstreifen
- 22: Längskante
- 24: Längskante
- 26: Stirnseite
- 28: Stirnseite
- 30: Einlage

## Patentansprüche

1. Therapeutisches Kopfband , dadurch gekennzeichnet, daß es ausschließlich aus natürlichen und weitgehend naturbelassenen Materialien besteht, daß es wenigstens abschnittsweise als Kissen (2) ausgebildet ist, daß dieses Kissen (2) mit im wesentlichen kugelförmigen Naturstoffen zur Erzeugung einer Massagewirkung gefüllt ist, und daß an einer oder beiden Oberflächen (16) des Kissens eine Tasche ausgebildet ist, in der eine oder mehrere Einlagen (30) auswechselbar angeordnet sind.

2. Therapeutisches Kopfband nach Anspruch 1, dadurch gekennzeichnet, daß die Naturstoffe Wärme und/oder Kälte speichern können.

3. Therapeutisches Kopfband nach Anspruch 1 oder 2, dadurch gekennzeichnet, daß die Naturstoffe beständig gegen Hitze von bis zu 150°C sind.

4. Therapeutisches Kopfband nach einem der Ansprüche 1 bis 3, dadurch gekennzeichnet, daß das Kissen (2) mit Hirseschalen gefüllt ist.

5. Therapeutisches Kopfband nach einem der Ansprüche 1 bis 4, dadurch gekennzeichnet, daß das Kissen (2) mit Hirseschalen und Torf gefüllt ist.

6. Therapeutisches Kopfband nach einem der Ansprüche 1 bis 4, dadurch gekennzeichnet, daß das Kissen (2) mit Kirschkernen gefüllt ist.

7. Therapeutisches Kopfband nach einem der Ansprüche 1 bis 6, dadurch gekennzeichnet, daß die Kissenfüllung (14) aromatisiert ist.

8. Therapeutisches Kopfband nach einem der Ansprüche 1 bis 7 , dadurch gekennzeichnet, daß die Tasche(n) (18) im wesentlichen die Oberfläche(n) (16) des Kissens (2) einnimmt (einnehmen).

9. Therapeutisches Kopfband nach einem der Ansprüche 1 bis 8, dadurch gekennzeichnet, daß die Einlage (30) aus Naturfasern mit hohem Feuchtigkeitsaufnahmevermögen besteht.

10. Verwendung des therapeutischen Kopfband nach einem der Ansprüche 1 bis 9 als Stirnkompresse insbesondere zur Behandlung von Migränekopfschmerzen.

11. Verwendung des therapeutischen Kopfband nach einem der Ansprüche 1 bis 9 als Ohrenkompresse insbesondere zur Behandlung von Ohrenentzündungen.

12. Verwendung des therapeutischen Kopfband nach einem der Ansprüche 1 bis 9 als Nasen- und/oder Nasennebenhöhlenkompresse.

13. Verwendung des therapeutischen Kopfbandes nach einem der Ansprüche 1 bis 9 als Nackenkompresse, insbesondere zur Behandlung von Nackenverspannungen und Kopfschmerzen
